Europäisches Patentamt

European Patent Office

Office européen des brevets

(11) Publication number: **0 081 753**
**B1**

# (12) EUROPEAN PATENT SPECIFICATION

(45) Date of publication of patent specification: **06.11.85**

(51) Int. Cl.⁴: **C 07 C 65/11, C 07 C 51/15**

(21) Application number: **82111139.0**

(22) Date of filing: **02.12.82**

(54) **Process for selective production of 2-hydroxynaphthalene-6-carboxylic acid.**

(30) Priority: **07.12.81 JP 195599/81**

(43) Date of publication of application:
**22.06.83 Bulletin 83/25**

(45) Publication of the grant of the patent:
**06.11.85 Bulletin 85/45**

(84) Designated Contracting States:
**BE CH DE FR GB IT LI NL**

(56) References cited:
EP-A-0 053 824
US-A-4 239 913
US-A-4 287 357

CHEMICAL ABSTRACTS, vol. 83, no. 13, 29th
September 1975, page 516, no. 113990e,
Columbus Ohio (USA);

(73) Proprietor: **Kabushiki Kaisha Ueno Seiyaku Oyo
Kenkyujo
2-31, Koraibashi
Higashi-ku Osaka (JP)**

(72) Inventor: **Ryuzo, Ueno
10-27, Nango-cho
Nishinomiya-shi Hyogo-ken (JP)**
Inventor: **Kazuyuki, Sakota
1798-7, Kobayashi Sumiyoshi-cho Higashinada-
ku
Kobe-shi Hyogo-ken (JP)**

(74) Representative: **Dr. E. Wiegand Dipl.-Ing. W.
Niemann Dr. M. Kohler Dipl.-Ing. J. Glaeser Dr.
H.-R. Kressin Patentanwälte
Herzog-Wilhelm-Strasse 16
D-8000 München 2 (DE)**

Courier Press, Leamington Spa, England.

## Description

This invention relates to an improvement in a process for the production of 2-hydroxynaphthalene-6-carboxylic acid by the reaction of potassium β-naphtholate with carbon dioxide.

2-Hydroxynaphthalene-6-carboxylic acid is an aromatic hydroxycarboxylic acid which, together with p-hydroxybenzoic acid, has recently attracted attention as a material for polymer production because a polyester derived from it and p-hydroxybenzoic acid is an excellent material for the production of fibers having high tensile properties and high heat resistance or other articles having high heat resistance.

Few literature references are available as to the method of synthesizing this aromatic hydroxycarboxylic acid, U.S. Patent No. 1,593,816 describes a method for synthesizing 2-hydroxynaphthalene-6-carboxylic acid by reacting potassium β-naphtholate in solid form with carbon dioxide. The U.S. Patent states that the reaction is carried out at 170 to 230°C for 8 hours, but fails to describe other reaction conditions, particularly the pressure of carbon dioxide. It also fails to disclose the proportion of by-product 2-hydroxynaphthalene-3-carboxylic acid. Probably, according to the method of this U.S. Patent, the desired product is not fully separated from the by-product 3-carboxylic acid or free β-naphthol. Investigations of the present inventors have shown that when the method of this U.S. Patent was reproduced, the yield of 2-hydroxynaphthalene-6-carboxylic acid was very low, i.e. less than 10%, and the yield of by-product 2-hydroxynaphthalene-3-carboxylic acid was more than about twice as large.

An improvement over this method was recently published as U.S. Patent NO. 4,287,357. This U.S. Patent discloses a method which comprises mixing a potassium base and 0.8 to 1.2 moles, per equivalent of the potassium base, of β-naphthol, dehydrating the mixture, and reacting the dehydrated mixture with carbon dioxide at a temperature of about 255 to about 280°C under a carbon dioxide pressure of 0.4 to 5.3 kg/cm²(G) until the ratio of 2-hydroxynaphthalene-6-carboxylic acid to 2 - hydroxynaphthalen - 3 - carboxylic acid reaches at least 2. This patent document describes an embodiment in which the steps of the above method are carried out in the presence of 1-isopropylnaphthalene and 2-isopropylnaphthalene. The yield of the 2-hydroxynaphthalene-6-carboxylic acid, based on potassium β-naphtholate, in this method is only less than 27%, and the patent fails to disclose anything about the continuous performance of the reaction between potassium β-naphtholate and carbon dioxide. Accordingly, this method is also unsuitable for the industrial mass production of 2-hydroxynaphthalene-6-carboxylic acid having a purity required as a material for polymer production.

In order to establish a method for the industrial mass-production of 2-hydroxynaphthalene-6-carboxylic acid from β-naphthol, the present inventors have studied the reaction of potassium β-naphtholate and carbon dioxide in a reaction medium. This work has finally led to the discovery that the ratio and yield of 2-hydroxynaphthalene-6-carboxylic acid produced can be increased, and this acid can be obtained substantially selectively, by selecting a carbon dioxide pressure suitable for the reaction temperature and continuously reacting the aforesaid materials while removing the free β-naphthol formed in the reaction system by using a reaction medium.

Thus, according to this invention, there is provided a process for the selective production of 2-hydroxynaphthalene-6-carboxylic acid, which comprises continuously reacting potassium β-naphtholate with carbon dioxide at a reaction temperature in the range of 230 to 350°C and under a carbon dioxide pressure suitable for the reaction temperature and selected within the range of 1 to 20 kg/cm²(G) while removing the by-product β-naphthol by using a reaction medium having a specific gravity of 0.6 to 1.5 and being capable of dissolving β-naphthol but substantially incapable of dissolving potassium β-naphtholate.

According to the process of this invention, 2-hydroxynaphthalene-6-carboxylic acid and 2-hydroxynaphthalene-3-carboxylic acid are obtained in a weight ratio of at least 97:3, and the yield of 2-hydroxynaphthalene-6-carboxylic acid reaches about 45% based on potassium β-naphtholate. Furthermore, since the reaction in accordance with this invention is carried out while removing β-naphthol, the decomposition of the reaction product is inhibited to reduce the formation of a tarry substance, and the total yield of the two acids based on the consumed β-naphthol reaches at least about 90%. In addition, according to this invention, an acid precipitating step and a separating step for obtaining the desired compound can be very much simplified.

The reaction medium used in this invention should be capable of dissolving β-naphthol but substantially incapable of dissolving potassium β-naphtholate and have a specific gravity at ordinary temperatures of 0.6 to 1.5, preferably 0.7 to 1.4, in order to perform the reaction continuously while removing the by-product β-naphthol.

Suitable reaction media are aliphatic, alicyclic or aromatic hydrocarbons or ethers having hydrocarbon groups derived from these hydrocarbons. Examples include light oil (gas soil), kerosene, gasoline, lubricating oils, white oil, alkylbenzenes, alkylnaphthalenes, diphenyl, diphenylalkanes, triphenyls, hydrogenated triphenyls, diphenyl ether, alkyl diphenyl ethers (such as ditolyl ether), and alkyl phenyl ethers. Preferred reaction media are those having a boiling point in the range of 150 to 400°C, particularly 180 to 400°C.

It is necessary that potassium β-naphtholate used in the reaction be fully dehydrated. It can be prepared in a customary manner from β-naphthol and an alkaline potassium compound. Potassium hydroxide, potassium carbonate, etc. can be used as the alkaline potassium compound, but potassium hydroxide is especially preferred. β-Naph-

thol can be used in an amount of 0.97 to 1.03 moles, preferably about 1.00 mole, per equivalent of the alkaline potassium compound. A thick aqueous solution of potassium β-naphtholate can be obtained, for example, by reacting β-naphthol with an aqueous solution of potassium hydroxide. Since it is necessary to render potassium β-naphtholate substantially anhydrous, the thick aqueous solution is heated to a temperature of at least 240°C, preferably 250 to 300°C, at atmospheric pressure or slightly reduced or elevated pressures in a stream of an inert gas such as nitrogen. Since potassium β-naphtholate has a melting point of about 235°C and is liquid at the aforesaid heating temperature, it can be dehydrated continuously or batchwise in the absence of a reaction medium. The absence of a reaction medium in the dehydrating step is advantageous because it obviates the need for using an intense stirring operation and apparatus for uniformly mixing the reaction medium and potassium β-naphtholate which have a larger difference in specific gravity. Furthermore, when no reaction medium is used in the dehydrating step, the reaction medium can be fed into the reaction mixture of potassium β-naphtholate and carbon dioxide in an accurate proportion at a suitable temperature. Hence, the heat of the reaction between potassium β-naphtholate and carbon dioxide can be easily removed, and temperature adjustment can be effected well whereby the yield and selectivity of the desired product can be increased. Of course, it is possible to carry out the reaction of β-naphthol with the alkaline potassium compound, and/or the step of dehydrating the resulting potassium β-naphtholate continuously or batchwise in the presence of the reaction medium.

The reaction of potassium β-naphtholate with carbon dioxide is carried out at a temperature in the range of 230 to 350°C, preferably 240 to 320°C, under a carbon dioxide pressure suitable for the reaction temperature and selected within the range of 1 to 20 kg/cm²(G), preferably 2 to 16 kg/cm²(G). Preferably, for example, the pressure of carbon dioxide is 1 to 4 kg/cm²(G) at a reaction temperature of 230 to 240°C; 4 to 7 kg/cm²(G) at a reaction temperature of 260°C; 7 to 10 kg/cm²(G) at a reaction temperature of 280°C; 10 to 13 kg/cm²(G) at a reaction temperature of 300°C; 13 to 16 kg/cm²(G) at a reaction temperature of 320°C; and 14 to 20 kg/cm²(G) at a reaction temperature of 320 to 350°C. The selection of such temperature and pressure conditions is due to the fact that high temperatures and low pressures are desirable for the selectivity of 2-hydroxynaphthalene-6-carboxylic acid and high pressures are desirable for its yield.

The reaction medium is used usually in an amount 0.5 times, preferably 0.5 to 10 times, particularly 1 to 5 times, the weight of potassium β-naphtholate. When a reaction medium is used also in the preparation of potassium β-naphtholate (i.e., in the reaction of β-naphthol with the alkaline potassium compound) and/or in the dehydration of potassium β-naphthol, the amount of the reaction medium is preferably larger by an amount which will azeotrope with water.

The reaction medium is capable of dissolving free β-naphthol in the reaction system but substantially incapable of dissolving potassium β-naphthol and has a specific gravity of 0.6 to 1.5. β-Naphthol acts to inhibit the formation of scaling or a coating on the liquid surface by the reaction product, but tends to decompose the 2-hydroxynaphthalene-6-carboxylic acid salt and other byproducts to tarry substances. For this reason, in the reaction of potassium β-naphtholate with carbon dioxide, it is necessary for increased yields and selectivities to perform the reaction while removing by-product β-naphthol out of the reaction system as a solution in the reaction medium thereby to avoid an increase in the concentration of the by-product β-naphthol. Since the reaction medium can be easily separated not only from potassium β-naphtholate but also from the 2-hydroxynaphthalene-6-carboxylic acid salt, β-naphthol can be continuously removed out of the reaction system as a solution in the reaction medium during the reaction by performing the continuous reaction with proper stirring.

The work-up of the reaction mixture is carried out as follows: After the reaction of potassium β-naphtholate and carbon dioxide, water is added to the reaction mixture, and the mixture is adjusted to pH 6.5—8 with an acid such as sulfuric acid or hydrochloric acid to liberate the unreacted potassium β-naphtholate as β-naphthol. Before or after this operation, the reaction medium layer is separated, and from the aqueous layer, a tarry layer containing β-naphthol and a resinous material is sedimented in liquid form and separated. The separated tarry layer is washed with water, and the washing is reused as a portion of the water to be added. The aforesaid aqueous layer is extracted with a liquid hydrophobic solvent at a temperature of not more than 110°C. Examples of the extracting solvent are hydrocarbons, halogenated hydrocarbons, ethers, ketones, and alcohols having at least 4 carbon atoms. The aqueous layer is extracted at 30 to 110°C using the extracting solvent in an amount 0.3 to 2 times the volume of the aqueous layer. Preferably, β-naphthol in the reaction medium layer is directly recycled. Or it is preferred to recover β-naphthol in the reaction medium layer and the extract as an aqueous solution of potassium β-naphtholate by the action of an aqueous solution of potassium hydroxide on it. β-Naphthol in the extract or the tarry layer can be recovered by reduced pressure distillation, etc. The recovered aqueous potassium β-naphtholate solution and β-naphthol can be recycled to the step of preparing the starting material for re-use.

Thereafter, the pH of the aqueous solution after extraction is adjusted to about 3 to 5, preferably about 3.5 to 4.5, and it is precipitated with an acid to separate 2-hydroxynaphthalene-6-carboxylic acid of high purity. Further precipitation of the mother liquor at a pH of about 1 to 3, preferably about 1.5 to 2.5, gives a mixture of 2-hydroxynaphthalene - 6 - carboxylic acid and 2 - hydroxynaphthalene-3-carboxylic acid. The mixture can be easily separated into the individual acids by, for example, washing it with an organic solvent or a hydrous organic solvent. Depending upon the end use of the final product, it is possible to subject the aqueous layer after the extraction directly to acid precipitation at a pH of not more than about 3. The acid precipitation step can be so simplified because the process of this invention can give 2-hydroxynaphthalene-6-carboxylic acid in an increased selectivity and an increased yield.

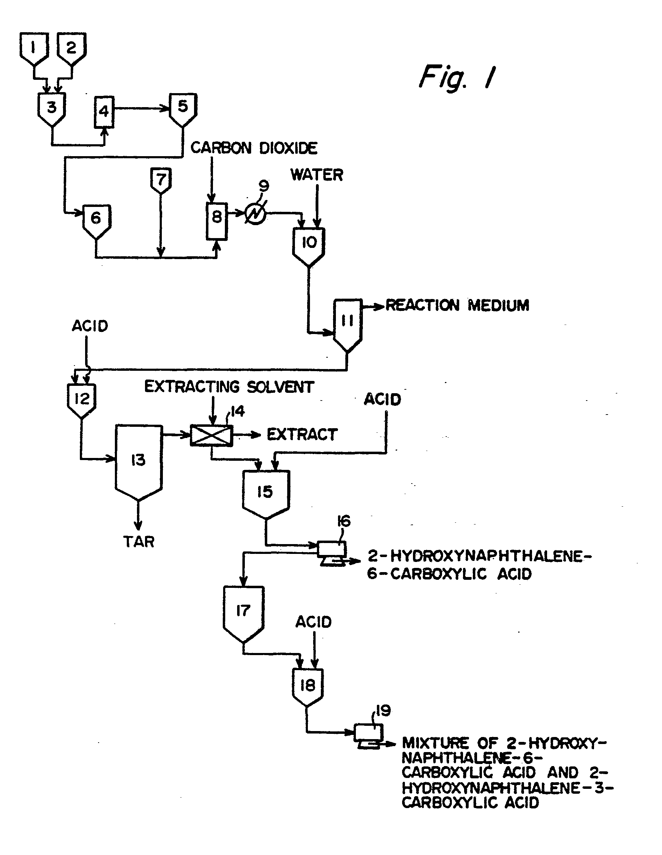

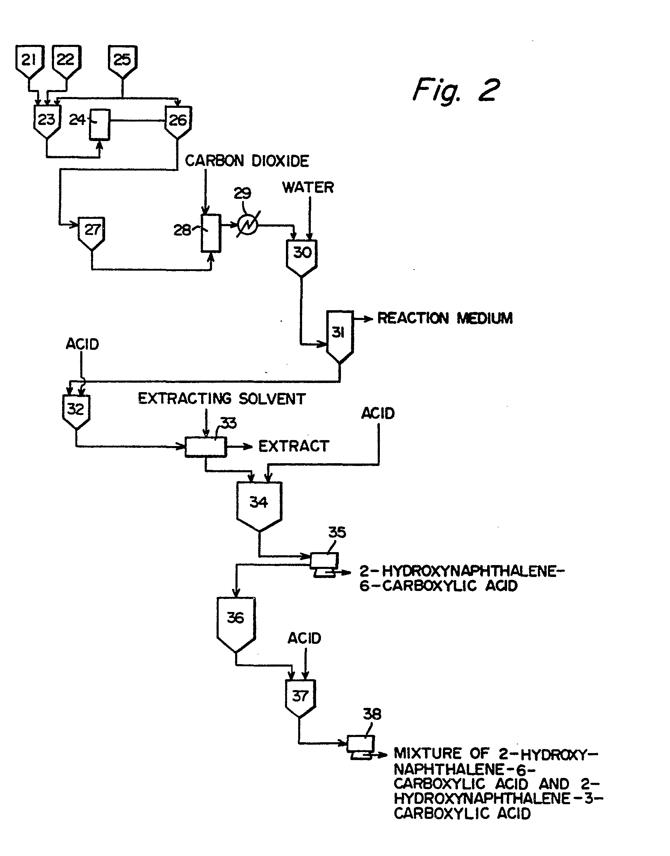

The present invention is described more specifically with reference to the accompanying drawings, Figures 1 and 2, which are diagrams showing the production of 2-hydroxynaphthalene-6-carboxylic acid from β-naphthol in accordance with preferred embodiments.

In Figure 1, β-naphthol placed in a reservoir tank 1 and a potassium hydroxide solution placed in a reservoir tank 2 are sent to a preparation tank 3 to give an aqueous solution of potassium β-naphtholate. The aqueous solution is sent to a concentrating tank 4 and then dehydrated in a dehydrating tank 5 to form substantially anhydrous potassium β-naphtholate. The potassium β-naphtholate placed in a reservoir tank 6 and a reaction medium placed in a separate reservoir tank 7 are sent to a reaction tank 8 having a proper stirring efficiency and reacted at the reaction temperature under the carbon dioxide pressure described hereinabove. The residence time is preferably 1 to 10 hours. The reaction mixture from the reaction tank 8 is cooled preferably in a heat-exchanger 9, and mixed with water with stirring in a water mixing tank 10. The mixture is then separated into a reaction medium layer and an aqueous layer in a separating tank 11. β-Naphthol can be recovered from the reaction medium layer (upper layer) by using a recovering device (not shown).

The aqueous layer (lower layer) from the separating tank 11 is adjusted to pH 6.5—8 by adding an acid in a pH adjusting tank 12 and then sent to a separating tank 13 where a tarry layer is sedimented in liquid form. β-Naphthol can be recovered from the tarry layer by, for example, using a vacuum distillation device (not shown). The upper layer in the separating tank 13 is sent to an extracting device (preferably centrifugal) extracting device 14 where it is extracted with a hydrophobic extracting solvent. β-Naphthol can be recovered from the solvent layer by using a recovering device (not shown). The aqueous layer from the extracting device 14 is sent to an acid precipitation tank 15 where it is precipitated with an acid at a pH of 3 to 5. The precipitate is separated by a centrifugal separator 16 to give substantially pure 2 - hydroxynaphthalene - 6 - carboxylic acid. The mother liquor is sent to an acid precipitation tank 18 through a reservoir tank 17, and precipitated with an acid at a pH of 1 to 3. The precipitate is separated by a centrifugal separator 19 to give a mixture of 2-hydroxynaphthalene-6-carboxylic acid and 2-hydroxynaphthalene-3-carboxylic acid. The mixture can be separated into the constituent acids by washing it with a dilute alcohol. Depending upon the end use of the desired product, the aqueous layer from the extraction device 14 may be directly subjected to acid precipitation at a pH of not more than 3 in the acid precipitation tank 15.

The β-naphthol and aqueous potassium β-naphtholate solution recovered from the reaction medium layer, the extracting solvent layer and the tarry layer are recycled to the step of preparing the starting material.

According to another embodiment shown in Figure 2, the process differs from that shown in Figure 1 in that a reservoir 25 is provided for feeding a reaction medium into a preparation tank 23 and/or a dehydration tank 26 so as to carry out the reaction of β-naphthol with an alkaline potassium compound and/or the dehydration of the resulting potassium β-naphtholate in the presence of the reaction medium, and that a pH adjusting tank 32 is connected to a centrifugal extracting device 33 and the separating tank 13 shown in Figure 1 is omitted. Much the same results are obtained as in Figure 1 by the operations shown in Figure 2.

According to the process of this invention, the yield of 2-hydroxynaphthalene-6-carboxylic acid based on potassium β-naphtholate usually reaches about 45% with a residence time of 1 to 10 hours, and the weight ratio of 2-hydroxynaphthalene-6-carboxylic acid to 2-hydroxynaphthalene-3-carboxylic acid in the reaction product reaches at least 97:3. Hence, the desired product can be produced substantially selectively. The total yield of the two acids based on the concumed β-naphthol and the ratio of β-naphthol recovered reach at least 90%.

As stated above, potassium β-naphtholate can be dehydrated in the absence of a reaction medium, in which case the stirring operation and the stirring device used in the dehydrating step can be simplified. Furthermore, by feeding a reaction medium, kept at a suitable temperature into the reaction system, the heat of the reaction between potassium β-naphtholate and carbon dioxide can be easily removed, and temperature can be well controlled, whereby the yield and selectivity of the desired product can be increased. As a result, the acid precipitation can be simplified in this invention to make it very easy to produce 2-hydroxynaphthalene-6-carboxylic acid continuously from β-naphthol. Thus, the process of this invention is very useful in industry.

The following non-limitative examples illustrate the present invention further.

Example 1

The reaction and work-up were carried out continuously using the apparatus shown in Figure 1.

β-Naphthol in reservoir tank 1 and a 48% aqueous solution of potassium hydroxide in reservoir tank 2 were sent to preparation tank 3 at a rate of 144 kg and 116.7 kg, respectively, per hour to give an aqueous solution of potassium β-naphtholate. The aqueous solution was sent to concentrating tank 4, and then dehydrated in dehydrating tank 5 at 265°C while introducing nitrogen at a rate of 75 Nm³/hr. The resulting substantially anhydrous, liquid potassium β-naphtholate was sent to reservoir tank 6 at a rate of 182 kg/hr. Light oil (boiling point 200 to 310°C) in reservoir tank 7 was fed to reaction tank 8 at a rate of 455 kg/hr and also potassium β-naphtholate was fed from reservoir tank 6 into reaction tank 8 at a rate of 182 kg/hr. The reaction tank 8 was kept at a carbon dioxide pressure of 9 kg/cm²(G), and potassium β-naphtholate and carbon dioxide were reacted there at 280°C. The residence time was 3 hours. The reaction mixture leaving the reaction tank 8 was cooled in heat exchanger 9, and mixed with 800 liters/hr of water with stirring in mixing tank 10. The mixture was adjusted to a temperature of 80°C, and sent to separating tank 11 where it was separated at 80°C into a light oil layer and an aqueous layer. β-Naphthol was recovered from the upper light oil layer by using a recovering device (not shown).

The lower aqueous layer was adjusted to pH 7.0 with dilute sulfuric acid in pH adjusting tank 12, and separated at 80°C in separating tank 13. β-Naphthol was recovered by a vacuum distillation device (not shown) from the lower tarry layer separated in separating tank 13. The upper layer in separating tank 13 was sent to centrifugal extracting device where it was extracted at 80°C with 500 liters/hr of toluene. The toluene layer was sent to a recovering device (not shown) to recover β-naphthol. The aqueous layer from extracting device 14 was sent to acid precipitation tank 15, and precipitated with dilute sulfuric acid at a pH of 3.8, and the precipitate was separated by centrifugal separator 16 to give 81.8 kg/hr of 2-hydroxynaphthalene - 6 - carboxylic acid as crystals. The mother liquor of centrifugation was sent to acid precipitation tank 18 through reservoir tank 17, and precipitated with dilute sulfuric acid at a pH of 2.0. The precipitate was separated by centrifugal separator 19 to give 5.1 kg/hr of crystals containing 2.5 kg/hr of 2-hydroxynaphthalene-6-carboxylic acid and 2.6 kg/hr of 2-hydroxynaphthalene-3-carboxylic acid. The crystals were washed with dilute methanol in a washing tank (not shown) to give the respective acids as pure products. Acid precipitation at a pH of 2.8 in acid precipitation tank 15 gave 86.1 kg/hr of crystals containing 84.4 kg/hr of 2-hydroxynaphthalene-6-carboxylic acid and 1.7 kg/hr of 2-hydroxynaphthalene-3-carboxylic acid.

The yield of 2-hydroxynaphthalene-6-carboxylic acid based on potassium β-naphtholate was 44.9%, and the total yield of the two acids was 46.3%. 70.0 kg of β-naphthol was recovered, and the total yield of the acids based on the consumed β-naphthol was 90.1% and the ratio of recovery of β-naphthol was 90.5%.

Example 2

In the apparatus shown in Figure 2, β-naphthol in reservoir tank 21 and a 48% aqueous solution of potassium hydroxide in reservoir tank 22 were fed into preparation tank 23 at a rate of 115.2 kg and 93.3 kg, respectively, per hour to give an aqueous solution of potassium β-naphtholate. The aqueous solution was sent to concentrating tank 24 where it was concentrated. The concentrated solution, together with 436.8 kg/hr of a hydrogenated triphenyl mixture (boiling point 310 to 360°C) as a reaction medium in reservoir tank 25, was sent to dehydrating tank 26 and dehyra-ted at 260°C while introducing nitrogen at a rate of 60 Nm³/hr. A dispersed mixture of substantially anhydrous liquid potassium β-naphtholate and 334.9 kg of the reaction medium was sent to reservoir tank 27 at a rate of 145.6 kg/hr. The mixture was sent to reaction tank 28 kept at a carbon dioxide pressure of 7 kg/cm²(G) at a rate of 480.5 kg/hr, and reacted with carbon dioxide at 270°C. The residence time was 4 hours. The reaction mixture leaving the reaction tank 28 was cooled by heat exchanger 29, and mixed with 640 liters/hr of water with stirring in water mixing tank 30. After adjusting the temperature of the mixture to 85°C, it was sent to separating tank 31, and separated at 85°C into a reaction medium layer and an aqueous layer. β-Naphthol was recovered from the upper reaction medium layer by using a recovering device (not shown).

The lower aqueous layer was adjusted to pH 6.8 with dilute sulfuric acid in pH adjusting tank 32, and sent to centrifugal extracting device 33 where it was extracted with 500 liters/hr of xylene at 85°C. The xylene layer was sent to a recovering device (not shown) to recover β-naphthol. The aqueous layer leaving extracting device 33 was sent to acid precipitation tank 34, and precipitated with dilute sulfuric acid at a pH of 4.0. The precipitate was separated by centrifugal separator 35 to give 60.8 kg/hr of 2-hydroxynaphthalene-6-carboxylic acid as crystals. The mother liquor of centrifugation was sent to acid precipitation tank 37 through reservoir tank 36, and precipitated with dilute sulfuric acid at a pH of 2.0. The precipitate was separated by centrifugal separator 38 to give 6.6 kg/hr of crystals containing 3.6 kg/hr of 2-hydroxynaphthalene-6-carboxylic acid and 3.0 kg/hr of 2 - hydroxynaphthalene - 3 - car-boxylic acid. When the crystals were washed with dilute methanol in a washing tank (not shown), the individual acids were obtained as pure products.

The yield of 2-hydroxynaphthalene-6-carboxylic acid based on potassium β-naphthol was 42.8%, and the total yield of the two acids was 44.8%. 57.6 kg of β-naphthol was recovered, and the total yield of the two acids based on the consumed β-naphthol was 89.6%, and the ratio of β-naphthol recovered was 90.0%.

Much the same results were obtained when the above procedure was repeated except that 1-phenyl-1-(2,3-dimethylphenyl)ethane (boiling point 292—306°C) or Dowtherm A (a mixture of 75% of diphenyl ether and 25% of diphenyl; boiling point 257°C) was used instead of the hydrogenated triphenyl mixture.

## Claims

1. A process for the selective production of 2-hydroxynaphthalene-6-carboxylic acid, which comprises continuously reacting potassium β-naphtholate with carbon dioxide at a reaction temperature in the range of 230 to 350°C and under a carbon dioxide pressure suitable for the reaction temperature and selected within the range of 1 to 20 kg/cm²(G), characterized in that the by-product β-naphthol is removed continuously by using a reaction medium having a specific gravity of 0.6 to 1.5 and being capable of dissolving β-naphthol but substantially incapable of dissolving potassium β-naphtholate and by performing the continuous reaction with proper stirring.

2. The process of claim 1 wherein the reaction of β-naphthol with an alkaline potassium compound and/or the dehydration of potassium β-naphtholate obtained by this reaction is carried out in the presence of the reaction medium, and then potassium β-naphtholate is reacted with carbon dioxide in the presence of the reaction medium.

3. The process of claim 1 wherein the reaction of β-naphthol with an alkaline potassium compound and/or the dehydration of potassium β-naphtholate obtained by this reaction is carried out in the absence of the reaction medium, and then potassium β-naphtholate is reacted with carbon dioxide in the presence of the reaction medium.

## Patentansprüche

1. Verfahren zur selektiven Herstellung von 2-Hydroxynaphthalin-6-carbonsäure durch kontinuierliche Umsetzung von Kalium-β-naphtholat mit Kohlenstoffdioxid bei einer Reaktionstemperatur im Bereich von 230 bis 350°C und bei einem für die Reaktionstemperatur geeigneten Kohlenstoffdioxiddruck, ausgewählt im Bereich von 1 bis 20 kg/cm²(G), dadurch gekennzeichnet, daß man das Nebenprodukt β-Naphthol kontinuierlich entfernt durch Verwendung eines Reaktionsmediums mit einem spezifischen Gewicht von 0,6 bis 1,5, das fähig ist, β-Naphthol zu lösen, aber im wesentlichen nicht fähig ist, Kalium-β-naphtholat zu lösen, und daß man die kontinuierliche Umsetzung unter entsprechendem Rühren durchführt.

2. Verfahren nach Anspruch 1, dadurch gekennzeichnet, daß man die Umsetzung von β-Naphthol mit einer alkalischen Kaliumverbindung und/oder die Dehydrierung des bei dieser Umsetzung erhaltenen Kalium-β-naphtholats durchführt in Gegenwart eines Reaktionsmediums, und daß man dann das Kalium-β-naphtholat mit Kohlenstoffdioxid in Gegenwart eines Reaktionsmediums umsetzt.

3. Verfahren nach Anspruch 1, dadurch gekennzeichnet, daß man die Umsetzung von β-Naphthol mit einer alkalischen Kaliumverbindung und/oder die Dehydrierung des bei dieser Reaktion erhaltenen Kalium-β-naphtholats durchführt in Abwesenheit eines Reaktionsmediums und daß man dann das Kalium-β-naphtholat mit Kohlenstoffdioxid in Gegenwart eines Reaktionsmediums umsetzt.

## Revendications

1. Un procédé de production sélective de l'acide 2-hydroxynaphtalène-6-carboxylique dans lequel on fait réagir en continu le β-naphtolate de potassium avec du dioxyde de carbone à une température de 230 à 350°C sous une pression relative du dioxyde de carbone appropriée à la température de réaction, comprise entre 1 et 20 bars, procédé caractérisé en ce que le β-naphtol formé comme sous produit est éliminé en continu au moyen d'un milieu réactionnel ayant une densité de 0,6 à 1,5 et pouvant dissoudre le β-naphtol mais pratiquement pas le β-naphtolate de potassium, et en ce que la réaction continue est effectuée avec une agitation appropriée.

2. Procédé selon la revendication 1 dans lequel la réaction du β-naphtol avec un composé alcalin de potassium, et/ou la déshydratation du β-naphtolate de potassium résultant de cette réaction, sont effectuées en présence du milieu réactionnel, puis on fait réagir le β-naphtolate de potassium avec le dioxyde de carbone en présence du milieu réactionnel.

3. Procédé selon la revendication 1 dans lequel la réaction du β-naphtol avec le composé alcalin de potassium, et/ou la déshydratation du β-naphtolate de potassium résultant de cette réaction, sont effectuées sans le milieu réactionnel, puis la réaction du β-naphtolate de potassium avec le dioxyde de carbone s'effectue en présence du milieu réactionnel.

*Fig. 1*

Fig. 2